(19) Europäisches Patentamt
European Patent Office
Office européen des brevets

(11) Veröffentlichungsnummer: **0 087 576**
**B1**

(12) # EUROPÄISCHE PATENTSCHRIFT

(45) Veröffentlichungstag der Patentschrift: **20.11.86**

(51) Int. Cl.⁴: **C 07 D 311/72**

(21) Anmeldenummer: **83100571.5**

(22) Anmeldetag: **22.01.83**

(54) **Verfahren zur Herstellung von Tocopherylacetat oder Tocopherylpropionat.**

(30) Priorität: **03.02.82 DE 3203487**

(43) Veröffentlichungstag der Anmeldung: **07.09.83 Patentblatt 83/36**

(45) Bekanntmachung des Hinweises auf die Patenterteilung: **20.11.86 Patentblatt 86/47**

(84) Benannte Vertragsstaaten: **CH DE FR GB LI NL**

(56) Entgegenhaltungen:
**DE - A - 2 208 795**
**DE - A - 2 606 830**
**DE - A - 2 743 920**

**Patent Abstracts of Japan Band 5, Nr. 140, 4. September 1981**
**Chemical Abstracts Band 67, Nr. 13, 25. September 1967, Columbus, Ohio, USA N.K. MAKHNOVSKII et al. "Initial compounds for synthesis of alpha-tocopheryl acetate" Seite 6030, Spalte 1, Abstract Nr. 64173z**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Schulz, Bernhard, Dr., Kurpfalzring 28, D-6830 Schwetzingen (DE)**
Erfinder: **Renauer, Erich, Dr., Mohnstrasse 44, D-6700 Ludwigshafen (DE)**

**EP 0 087 576 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein verbessertes Verfahren zur Herstellung von Tocopherylacetat

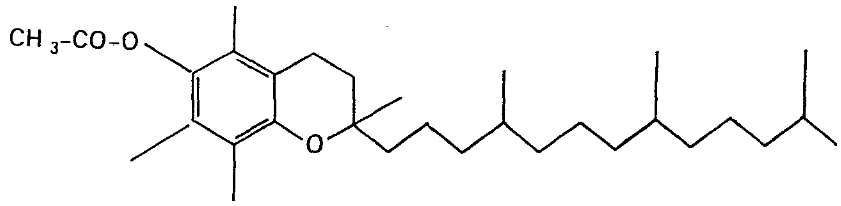

durch Umsetzen von Trimethylhydrochinon mit Phytol oder Isophytol in Gegenwart eines Protonendonators sowie von Zinkchlorid und anschliessende Veresterung des Tocopherols mit Essigsäureanhydrid oder Propionsäureanhydrid.

Dieses Verfahren, sieht man von der erfindungsgemässen Verbesserung ab, ist im Prinzip sowie in mehreren Varianten bekannt, lässt jedoch in den bisher bekannten Ausführungsformen zu wünschen übrig.

Nach Beispiel 1 der US-PS 3 444 213 nimmt man die Kondensation von Trimethylhydrochinon mit Isophytol in Gegenwart von Eisessig und $BF_3$-Etherat vor, trennt die flüchtigen Bestandteile weitgehend vom Reaktionsgemisch ab und erhitzt das rohe Tocopherol mit Essigsäureanhydrid und Pyridin. Diese Verfahrensweise ist relativ umständlich und energieaufwendig, erfordert die Mitverwendung von Pyridin und führt trotzdem nur zu einer Ausbeute an Tocopherylacetat von 87%, die wegen der teuren Einsatzstoffe unbefriedigend ist.

Ähnlich verhält es sich mit dem Verfahren der US-PS 3 708 505, bei welchem man die Kondensation von Trimethylhydrochinon mit Isophytol in Gegenwart einer Säure sowie von Zinkchlorid und von Benzol als Verdünnungsmittel vornimmt und das Umsetzungsgemisch anschliessend bei Siedetemperatur der Veresterungsreaktion mit Essigsäureanhydrid unterwirft. Je nach Art der Säure erhält man hier Roh-Ausbeuten an Tocopherylacetat zwischen 68 und 93,6%. Unter Berücksichtigung des Reinheitsgrades, der zwischen 64 und 89,5% liegt, sind die Ausbeuten an reinem Produkt jedoch entsprechend geringer. Weiterhin ist es nachteilig, dass die Veresterung 6 Stunden bei Siedehitze in Anspruch nimmt.

Aus der JP-PS 14 99 84 ist es ferner bekannt, Tocopherylacetat durch Umsetzen von Tocopherol mit Essigsäureanhydrid in Gegenwart von Chlorwasserstoff und Zinkstaub oder von Zinkchlorid herzustellen. Diese Reaktion verläuft adiabatisch unter Selbsterwärmung von Raumtemperatur auf etwa 60°C, nimmt etwa 1 bis 2 Stunden in Anspruch und liefert eine Esterausbeute von 96%, bezogen auf das eingesetzte D,L-α-Tocopherol.

Der Erfindung lag nun die Aufgabe zugrunde, Tocopherylacetat, ausgehend von Trimethylhydrochinon und Phytol oder Isophytol auf einfachere und wirtschaftlichere Weise herzustellen als bisher.

Es wurde nun überraschenderweise gefunden, dass die Acetylierung von Tocopherol mit Essigsäureanhydrid in Gegenwart von Chlorwasserstoff und Zinkstaub oder Zinkchlorid auch dann in kurzer Zeit und ohne Wärmezufuhr von aussen praktisch vollständig abläuft, wenn man nicht von isoliertem und getrocknetem α-Tocopherol ausgeht, sondern wenn man gleich das bei der Umsetzung von Trimethylhydrochinon mit Phytol oder Isophytol in Gegenwart eines Protonendonators, vorzugsweise Chlorwasserstoff, sowie von Zinkchlorid und eines flüssigen Kohlenwasserstoffs anfallende zweiphasige dunkelgefärbte Reaktionsgemisch nach dem Abkühlen mit Essigsäureanhydrid reagieren lässt. Nach beendeter Essigsäureanhydridzugabe ist die Acetylierung praktisch vollständig. Das in guter Ausbeute erhaltene Tocopherylacetat fällt hierbei überraschenderweise in sehr reiner Form an.

Ähnlich gute Erfolge ergaben sich bei der Herstellung von Tocopherylpropionat bei der Umsetzung des beschriebenen Reaktionsgemisches mit Propionsäureanhydrid.

Gegenstand der Erfindung ist demgemäss ein Verfahren zur Herstellung von Tocopherylacetat oder Tocopherylpropionat durch Umsetzen von Trimethylhydrochinon mit Phytol oder Isophytol in Gegenwart eines Protonendonators sowie von Zinkchlorid und eines flüssigen Kohlenwasserstoffes als Lösungsmittel, sowie gegebenenfalls in Gegenwart geringer Mengen einer Stickstoffbase, anschliessende Veresterung des erhaltenen Tocopherols und übliche Aufarbeitung des Veresterungsgemisches auf Tocopherylacetat oder Tocopherylpropionat, welches dadurch gekennzeichnet ist, dass man das bei Umsetzung von Trimethylhydrochinon mit Phytol bzw. Isophytol anfallende Reaktionsgemisch nach Abkühlung bei Temperaturen von 0 bis 60°C mit Essigsäureanhydrid bzw. Propionsäureanhydrid reagieren lässt.

Besonders vorteilhaft gestaltet sich das erfindungsgemässe Verfahren, wenn man ein bei Umsetzung von Trimethylhydrochinon mit Phytol bzw. Isophytol unter Wasserauskreisen anfallendes Reaktionsgemisch bei Temperaturen von 0 bis 60°C mit Essigsäureanhydrid bzw. Propionsäureanhydrid reagieren lässt.

Den ersten Verfahrensschritt nimmt man zweckmässigerweise in der Weise vor, dass man eine Mischung aus Trimethylhydrochinon, dem Protonendonator, Zinkchlorid und dem Kohlenwasserstoff bei 80 bis 130°C allmählich mit Phytol

oder Isophytol versetzt und das Gemisch etwa 1 bis 10 Stunden unter intensivem Vermischen in diesem Temperaturbereich hält.

Das Molverhältnis von Trimethylhydrochinon zu Phytol bzw. Isophytol beträgt hierbei vorzugsweise 0,9:1 bis 1,1:1 und das von Trimethylhydrochinon zum Zinkchlorid 20:1 bis 1:1.

Pro Mal (152 g) Trimethylhydrochinon verwendet man zweckmässigerweise etwa 0,5 bis 2 Liter des Kohlenwasserstoffs als Lösungsmittel.

Als Protonendonatoren kommen starke Mineralsäure, wie Salzsäure, Chlorwasserstoff oder Schwefelsäure, stark saure Salze, wie Natriumhydrogensulfat sowie starke organische Säuren wie p-Toluolsulfonsäure oder Trifluoressigsäure, vorzugsweise Salzsäure oder Chlorwasserstoff, in Betracht.

Geeignete Lösungsmittel sind Kohlenwaserstoffe wie Hexan, Heptan, Cyclohexan, Benzol oder Toluol.

Da die Kondensation des Trimethylhydrochinons mit Phytol bzw. Isophytol unter Wasserabspaltung verläuft, empfiehlt es sich, das im Reaktionsgemisch vorhandene und das entstehende Wasser während der Kondensation laufend aus dem Umsetzungsgemisch durch Auskreisen zu entfernen. Dies ist zwar nicht unbedingt notwendig, da die Reaktion auch ohne Wasserauskreisung glatt verläuft, jedoch benötigt man dann im anschliessenden Acylierungsschritt grössere Mengen an Carbonsäureanhydrid.

Weiterhin kann es sich empfehlen, die Umsetzung in an sich bekannter Weise in Gegenwart geringer Mengen einer Stickstoffbase, wie Ammoniak oder eines organischen Amins, z.B. von Stearylamin oder aber mit einem Phytol oder Isophytol vorzunehmen, das mit Ammoniak oder einem organischen Amin bei 20 bis 120°C behandelt worden ist (vgl. US-PS 4 191 692 bzw. DE-PS 2 606 830).

Nach beendeter Kondensation lässt man das erhaltene Reaktionsgemisch abkühlen, versetzt es bei Temperaturen von 0 bis 60°C mit Essigsäureanhydrid bzw. Propionsäureanhydrid, lässt gegebenenfalls noch 0,5 bis 3 Stunden bei 0 bis 60°C nachreagieren und arbeitet es sodann wie üblich auf das Tocopherylacetat oder Tocopherylpropionat auf, indem man es z.B. mit Wasser oder wässrigem Methanol extrahiert und aus der verbleibenden Kohlenwasserstoffphase das Tocopherylsalz isoliert. Die Acylierung verläuft unter Wärmeentwicklung, so dass es im allgemeinen nicht erforderlich ist, dem Reaktionsgemisch Wärme von aussen zuzuführen.

Mit Vorteil gibt man das Carbonsäureanhydrid unter Wasserkühlung bei 20 bis 50°C zu. Man kann die Acylierung auch bei tieferen Temperaturen, beispielsweise bei 0°C durchführen. Das bringt jedoch im allgemeinen keine wesentlichen Vorteile und man muss das Reaktionsgemisch vorher auf die tiefen Temperaturen abkühlen.

Auch für den Acylierungsschritt ist es überraschenderweise nicht unbedingt notwendig, das bei der Kondensation von Trimethylhydrochinon mit Phytol bzw. Isophytol gebildete Wasser während oder nach der Kondensation aus dem Reaktionsgemisch zu entfernen. Man muss dem Reaktionsgemisch lediglich eine dem enthaltenen Wasser äquimolare Menge Carbonsäureanhydrid zusätzlich zusetzen. Für trockene Reaktionsgemische benötigt man im allgemeinen 1 bis 1,5 Mol, vorzugsweise, 1,1 bis 1,3 Mol, Carbonsäureanhydrid pro Mol ursprünglich eingesetztem Trimethylhydrochinons, für Reaktionsgemische, aus denen das Kondensationswasser nicht entfernt wurde, dementsprechend 2 bis 3 Mol, vorzugsweise 2,1 bis 2,5 Mol. Grössere Mengen an Carbonsäureanhydrid können zwar verwendet werden, bringen aber keinen wirtschaftlichen Vorteil mehr. Auch bei Anwesenheit des Kondensationswassers im Reaktionsgemisch ist die Acylierung bereits direkt nach der Zugabe des letzten Anhydrids praktisch vollständig, d.h. der Gehalt an freiem Tocopherol ist kleiner als 0,1% (gemessen mit Hochdrucksäulenchromatographie).

Im übrigen liegt es im Wesen des erfindungsgemässen Verfahrens, dass das bei der Kondensation gebildete Tocopherol nicht isoliert zu werden braucht, und dass man das bei der Kondensation anfallende Reaktionsgemisch ohne weitere Reinigungs- oder Verfahrensschritte der Veresterung unterwerfen kann. Überraschenderweise ist das bei dem erfindungsgemässen Verfahren erhaltene Tocopherylsalz dennoch sehr rein. Das nach den bisherigen Verfahren übliche Vorhandensein von schwer abtrennbaren Carbonsäureanhydridresten im Produkt entfällt. Selbst aus Reaktionsansätzen in Heptan, bei denen bei der Kondensation mit einem Überschuss an Trimethylhydrochinon gearbeitet wurde, erhält man reines Tocopherylsalz, da das Trimethylhydrochinon beispielsweise bei der Umsetzung mit Essigsäureanhydrid vollständig das Trimethylhydrochinondiacetat bildet, welches durch Extraktion mit wässrigem Methanol aus der Hexanlösung leicht extrahierbar ist. Das ist sehr wichtig, da eine destillative Trennung von Tocopherylacetat und Trimethylhydrochinondiacetat technisch ausserordentlich problematisch wäre.

Das erfindungsgemässe Verfahren liefert das Tocopherylacetat auf einfache Weise und in sehr guter Qualität in Ausbeuten von 94 bis 98%. Der Gehalt an freiem Tocopherol liegt unter 0,1%.

Beispiel 1

a) Eine mit Chlorwasserstoffgas gesättigte Mischung aus 30,4 g (0,2 Mol) Trimethylhydrochinon, 5 g Zinkchlorid, 0,5 g Stearylamin und 250 ml Heptan wurde unter Aufrechterhaltung der Sättigung mit HCl zum Sieden (98°C) erhitzt, im Laufe einer Stunde mit 62,8 g (0,21 mol) Isophytol versetzt und noch eine weitere Stunde bei dieser Temperatur gehalten.

b) Das so erhaltene zweiphasige, sehr dunkel gefärbte Reaktionsgemisch wurde auf Raumtemperatur abkühlen lassen, danach mit 47 g (0,46 mol) Essigsäureanhydrid versetzt, wobei durch Kühlung die Temperatur bei 40°C gehalten wurde. Nach dem Abkühlen auf Raumtemperatur wurde das Reaktionsgemisch dreimal mit je

200 ml einer Mischung aus Methanol und Wasser im Volumenverhältnis 1:1 extrahiert. Die verbleibende Heptanphase wurde zur Trockene eingeengt. Man erhielt 96,1 g hellgelbes dl- -Tocopherylacetat mit einer Reinheit von 93,0% (USP XX). Der Gehalt an freiem Tocopherol betrug nach HPLC weniger als 0,1 Gew.%. Die Ausbeute betrug 94,5%, bezogen auf Trimethylhydrochinon. Durch Destillation erhält man hieraus Tocopherylacetat in einer Reinheit von 98% (U.S.P. XX).

Beispiel 2

Man arbeitet wie in Beispiel 1 beschrieben, jedoch unter zusätzlichem Auskreisen des sich bildenden Reaktionswassers in der ersten Verfahrensstufe. Durch Zugabe von 27 g (0,27 mol) Essigsäureanhydrid anstelle von 47 g in Beispiel 1 wurde acetyliert. Das Tocopherylacetat fiel in einer Reinheit von 94% und in einer Ausbeute von 98% (USPXX) an.

Beispiel 3

Es wurde gearbeitet wie in Beispiel 1a) beschrieben. Das erhaltene Reaktionsgemisch wurde dann statt auf Raumtemperatur auf 0°C gekühlt. Innerhalb von 20 Minuten wurden bei 0°C unter Rühren 47 g (0,46 mol) Essigsäureanhydrid zugegeben. Anschliessend wurde dreimal mit je 200 ml einer Mischung aus Methanol und Wasser (1:1) extrahiert und die verbleibende Heptanphase zur Trockene eingeengt. Man erhielt 96 g Tocopherylacetat mit einer Reinheit von 94%. Dies entspricht einer Ausbeute von 95%. Die Reinheit des destillierten Produktes betrug 97%.

Beispiel 4

Es wurde gearbeitet wie in Beispiel 1a) beschrieben, jedoch wurden anstelle von 30,4 g (0,2 mol) 38 g (0,25 mol) Trimethylhydrochinon eingesetzt. Das erhaltene Reaktionsgemisch wurde bei Raumtemperatur mit 57,2 g (0,56 mol) Essigsäureanhydrid versetzt, wobei die Temperatur auf 55°C anstieg. Anschliessend wurde dreimal mit 90%igem wässrigem Methanol und einmal mit 75%igem Methanol extrahiert. Man erhielt 95,8 g Tocopherylacetat mit einem Gehalt von 94,9%. Dies entspricht einer Ausbeute von 96,2%, bezogen auf das eingesetzte 97%ige Isophytol. Das Tocopherylacetat liess sich problemlos destillieren, da es kein Trimethylhydrochinondiacetat mehr enthielt. Der Gehalt des destillierten Produkts betrug 97,2% (U.S.P. XX).

Beispiel 5

Es wurde gearbeitet wie in Beispiel 1a) beschrieben. Das bei dieser Kondensation erhaltene zweiphasige, dunkel gefärbte Reaktionsgemisch wurde auf Raumtemperatur abgekühlt, danach mit 59,9 g (0,46 mol) Propionsäureanhydrid versetzt, wobei die Temperatur auf 50°C anstieg. Anschliessend wurde das Reaktionsgemisch auf Raumtemperatur abkühlen gelassen, dreimal mit je 200 ml einer Methanol-Wasser-Mischung (Volumensverhältnis 1:1) extrahiert. Die verbleibende Heptanphase wurde zur Trockene eingeengt. Man erhielt 101,2 g hellgelbes dl-α-Tocopherylpropionat mit einer Reinheit von 90,5% (Gaschromatographie). Der Gehalt an freiem Tocopherol betrug nach HPLC weniger als 0,1 Gew.%. Die Ausbeute betrug 94,1%, bezogen auf eingesetztes Trimethylhydrochinon. Durch Destillation erhält man hieraus Tocopherylpropionat in einer Reinheit von 95,2%.

**Patentansprüche**

1. Verfahren zur Herstellung von Tocopherylacetat oder Tocopherylpropionat durch Umsetzen von Trimethylhydrochinon mit Phytol oder Isophytol in Gegenwart eines Protonendonators sowie von Zinkchlorid und einer flüssigen Kohlenwasserstoffs als Lösungsmittel, sowie gegebenenfalls in Gegenwart geringer Mengen einer Stickstoffbase, anschliessende Veresterung des erhaltenen Tocopherols und übliche Aufarbeitung des Veresterungsgemisches auf Tocopherylacetat oder Tocopherylpropionat, dadurch gekennzeichnet, dass man das bei Umsetzung von Trimethylhydrochinon mit Phytol bzw. Isophytol anfallende Reaktionsgemisch nach Abkühlung bei Temperaturen von 0 bis 60°C mit Essigsäureanhydrid bzw. Propionsäureanhydrid reagieren lässt.

2. Verfahren gemäss Anspruch 1, dadurch gekennzeichnet, dass man ein bei Umsetzung von Trimethylhydrochinon mit Phytol bzw. Isophytol unter Wasserauskreisen anfallendes Reaktionsgemisch bei Temperaturen von 0 bis 60°C mit Essigsäureanhydrid bzw. Propionsäureanhydrid reagieren lässt.

**Claims**

1. A process for the preparation of tocopheryl acetate or tocopheryl propionate by reacting trimethylhydroquinone with phytol or isophytol in the presence of a proton donor and zinc chloride and of a liquid hydrocarbon as the solvent, and in the presence or absence of small amounts of a nitrogen base, esterifying the tocopherol obtained and working up the esterification mixture in a conventional manner to give tocopheryl acetate or tocopheryl propionate, wherein the mixture obtained in the reaction of trimethylhydroquinone with phytol or isophytol is cooled and then reacted with acetic anhydride or propionic anhydride at from 0 to 60°C.

2. A process as claimed in claim 1, wherein a mixture obtained by reacting trimethylhydroquinone with phytol or isophytol while separating off water is reacted with acetic anhydride or propionic anhydride at from 0 to 60°C.

**Revendications**

1. Procédé de préparation d'acétate de tocophérol ou de propionate de tocophérol par réaction de triméthylhydroquinone avec le phytol ou l'isophytol en présence d'un donneur de protons,

ainsi que de chlorure de zinc et d'un hydrocarbure liquide servant de solvant et, le cas échéant, en présence de faibles quantités d'une base azotée, puis par estérification du tocophérol obtenu et traitement ultérieur usuel du mélange d'estérification pour l'obtention de l'acétate de tocophérol ou du propionate de tocophérol, caractérisé en ce qu'après refroidissement, on fait réagir le mélange réactionnel formé par la réaction de triméthylhydroquinone et de phytol ou d'isophytol avec l'anhydride acétique ou l'anhydride acétique ou l'anhydride propionique à des températures de 0 à 60°C.

2. Procédé selon la revendication 1, caractérisé en ce qu'on fait réagir le mélange réactionnel formé par la réaction de triméthylhydroquinone et de phytol ou d'isophytol avec élimination de l'eau du circuit, avec l'anhydride acétique ou l'anhydride propionique à des températures de 0 à 60°C.